(19) Europäisches Patentamt
European Patent Office
Office européen des brevets

(11) Veröffentlichungsnummer : **0 469 423 B1**

(12) # EUROPÄISCHE PATENTSCHRIFT

(45) Veröffentlichungstag der Patentschrift : **15.06.94 Patentblatt 94/24**

(51) Int. Cl.[5] : **C07C 259/06,** A01N 37/30

(21) Anmeldenummer : **91112211.7**

(22) Anmeldetag : **20.07.91**

(54) **Oxalyl-bishydroxamsäurederivate, ihre Herstellung und sie enthaltende Mittel zur Regulierung des Pflanzenwachstums.**

(30) Priorität : **31.07.90 DE 4024283**

(43) Veröffentlichungstag der Anmeldung : **05.02.92 Patentblatt 92/06**

(45) Bekanntmachung des Hinweises auf die Patenterteilung : **15.06.94 Patentblatt 94/24**

(84) Benannte Vertragsstaaten : **BE CH DE DK ES FR GB IT LI NL**

(56) Entgegenhaltungen :
**CHEMICAL ABSTRACTS, vol. 106, no. 11, 16. März 1987, Columbus, Ohio,US; abstract no. 83981B, Seite 552 ;**
**BERICHTE DER DEUTSCHEN CHEMISCHEN GESELLSCHAFT. Bd. 27, 1894, WEINHEIM DE- Seiten 1105 - 1114; W. LOSSEN: 'ÜBER DIE EINWIRKUNG VON HYDROXYLAMIN UNDA-THOXYLAMIN AUF OXALÄTHER'**

(73) Patentinhaber : **BASF Aktiengesellschaft**
**Carl-Bosch-Strasse 38**
**D-67063 Ludwigshafen (DE)**

(72) Erfinder : **Rentzea, Costin, Dr.**
**Richard-Kuhn-Strasse 1-3**
**W-6900 Heidelberg (DE)**
Erfinder : **Keil, Michael, Dr.**
**Fontanestrasse 4**
**W-6713 Freinsheim (DE)**
Erfinder : **Harreus, Albrecht, Dr.**
**Teichgasse 13**
**W-6700 Ludwigshafen (DE)**
Erfinder : **Rademacher, Wilhelm, Dr.**
**Austrasse 1**
**W-6703 Limburgerhof (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Oxalyl-bishydroxamsäurederivate der allgemeinen Formel I

$R^1$-ONH-CO-CO-NHO-$R^2$ I,

in der die Substituenten folgende Bedeutung haben:
$R^1$ und $R^2$ unabhängig voneinander
$C_1$-$C_{20}$-Alkyl, $C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen oder einen Phenylring tragen können;
monocyclisches oder polycyclisches $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Phenylring tragen können;
Phenyl, Phenyl-$C_1$-$C_4$-alkyl oder Phenyl-$C_2$-$C_6$-alkenyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio,
wobei $R^1$ und $R^2$ nicht gleichzeitig Methyl oder Ethyl bedeuten sowie deren landwirtschaftlich brauchbaren Salze.

Außerdem betrifft die Erfindung Verfahren zur Herstellung dieser Verbindungen, sie enthaltende Mittel und Verfahren zur Verwendung von Oxalylbishydroxamsäurederivaten der allgemeinen Formel IA,

$R^1$-ONH-CO-CO-NHO-$R^2$ IA

in der die Substituenten folgende Bedeutung haben:
$R^1$ und $R^2$ unabhängig voneinander
$C_1$-$C_{20}$-Alkyl, $C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen oder einen Phenylring tragen können;
monocyclisches oder polycyclisches $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Phenylring tragen können;
Phenyl, Phenyl-$C_1$-$C_4$-alkyl oder Phenyl-$C_2$-$C_6$-alkenyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio und deren landwirtschaftlich brauchbaren Salze
als Pflanzenwachstumsregulatoren.

Oxalylhydroxamsäurederivate sind aus der Literatur als Synthesezwischenprodukte bekannt. Außerdem ist die Herstellung von Oxalyl-bisethylhydroxamsäure bekannt (Chem. Ber., 27, 1111 (1984); Chem. Ztg. 110 (3), 127 (1986) ≙ CA 106: 83891 b).

Aufgabe der vorliegenden Erfindung waren neue wirksame Pflanzenwachstumsregulatoren.

Demgemäß wurden die eingangs definierten Oxalyl-bishydroxamsäurederivate I gefunden. Außerdem wurden Verfahren zur Herstellung dieser Oxalyl-bishydroxamsäurederivate, sie enthaltende Mittel und Verfahren zur Verwendung der Verbindungen IA als Pflanzenwachstumsregulatoren gefunden.

Die Oxalyl-bishydroxamsäurederivate I sind auf verschiedenen Wegen zugänglich. Besonders vorteilhaft erhält man sie nach einem der im nachfolgenden beschriebenen Verfahren A und B.

Verfahren A:

Man erhält die Oxalyl-bishydroxamsäurederivate der Formel I beispielsweise dadurch, daß man einen Oxalylhydroxamsäurederivate der allgemeinen Formel II, in an sich bekannter Weise in einem inerten organischen Lösungsmittel mit einem Hydroxylaminderivat der allgemeinen Formel III, umsetzt.

$$R^1\text{-ONH-CO-CO-OCH}_3 \quad + \quad R^2\text{-ONH}_2 \quad \text{--->} \quad R^1\text{-ONH-CO-CO-NHO-}R^2$$

II III I

Die Umsetzung erfolgt im allgemeinen bei Temperaturen von 0 bis 100°C, vorzugsweise von 20 bis 90°C.

Als Lösungsmittel eignen sich beispielsweise Halogenkohlenwasserstoffe, insbesondere Chlorkohlenwasserstoffe, z.B. 1,1,2,2-Tetrachlorethylen, oder 1,1,2,2-Tetrachlorethan, Dichlorpropan, Methylenchlorid,

Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Di-isobutylether, Diisoamylether, Diisopropylether, Anisol, Phenethol, Cyclohexylmethylether, Diethylether, Ethylenglykoldimethylether, Tetrahydrofuran, Dioxan, Thioanisol; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Als Lösungsmittel können auch die Verbindungen der Formel I im überschuß verwendet werden. Zweckmäßig verwendet man das Lösungsmittel in einer Menge von 100 bis 2 000 Gew.-%, vorzugsweise von 200 bis 700 Gew.-%, bezogen auf Ausgangsstoff II.

Als Basen kommen beispielsweise Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-4-aminopyridin, N,N-Diethyl-4-aminopyridin, N,N-Dipropyl-4-aminopyridin, N-Methylpyrrolidin, N-Ethylpyrrolidin, N-Methylpiperidin, N-Ethylpiperidin, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, beta-Picolin, Isochinolin, Pyrimidin, Acridin, N,N,N,',N'-Tetramethylethylendiamin, N,N,N'-N'-Tetraethylethlyendiamin, Chinoxalin, N-Propyldiisopropylamin, N,N-Dimethylcyclohexylamin, 2,6-Lutidin, 2,4-Lutidin, Triethylendiamin in Betracht.

Zweckmäßig verwendet man die Base in stöchiometrischen Mengen, im überschuß oder Unterschuß von jeweils bis zu 20 Mol.%, bezogen auf den Ausgangsstoff II.

Außerdem kann es von nutzen sein, die Umsetzung in gegenwart eines Katalysators durchzuführen.

Als Reaktionsbeschleuniger kommen vorzugsweise 4-Dimethylaminopyridin und 4-Pyrrolidinopyridin in Betracht (J. Cossy et al., Synthesis, 753f (1989)).

Verfahren B:

Man erhält die Verbindungen der Formel I in denen $R^1$ und $R^2$ die gleiche Bedeutung haben beispielsweise auch dadurch, daß man ein Oxalylhalogenid der allgemeinen Formel IV, in an sich bekannter Weise in einem inerten organischen Lösungsmittel in Gegenwart eine Base mit einem Hydroxylamin der allgemeinen Formel III umsetzt.

$$\underset{IV}{\text{Hal-CO-CO-Hal}} + \underset{III}{R^1\text{-ONH}_2} \text{ ---> } \underset{I}{R^1\text{-ONH-CO-CO-NHO-}R^1}$$

Hal in der Formel IV steht für Halogenatome wie Fluor, Chlor, Brom und Jod, vorzugsweise für Chlor oder Brom.

Die Umsetzung erfolgt im allgemeinen bei Temperaturen von -20 bis 100°C, vorzugsweise von 15 bis 80°C.

Als Lösungsmittel eignen sich beispielsweise die vorstehend bei Verfahren A genannten. Insbesondere kommen die folgenden in Betracht: Essigsäure, Essigsäureethylester, Methylenchlorid, Toluol, Chlorbenzol, Tetrahydrofuran und Dioxan.

Als Basen kommen in diesem Verfahren neben den vorstehend genannten Kaliumacetat und Natriumacetat in Betracht.

Im Hinblick auf die bestimmungsgemäße Verwendung der Verbindungen IA in wachstumsregulierenden

Mitteln kommen als Substituenten folgende Reste in Betracht:

$R^1$ und $R^2$ unabhängig voneinander

$C_1$-$C_{20}$-Alkyl, vorzugsweise verzweigtes oder unverzweigtes $C_1$-$C_6$-Alkyl wie Methyl, Ethyl, Propyl, 1-Methylethyl, Butyl, 1-Methyl-propyl, 2-Methyl-propyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethyl-butyl, 2,3-Dimethylbutyl, 3,3-Dimethylbutyl, 1-Ethyl- butyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere verzweigtes oder unverzweigtes $C_1$-$C_4$-Alkyl wie Methyl, Ethyl, Propyl, 1-Methylethyl, Butyl, 1-Methyl-propyl, 2-Methylpropyl und 1,1-Dimethylethyl;

$C_3$-$C_{18}$-Alkenyl, besonders $C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl; oder

$C_3$-$C_8$-Alkinyl, besonders $C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl und 2-Butinyl;

wobei diese Gruppen ein bis fünf Halogenatome wie Fluor, Chlor, Brom und Jod, vorzugsweise Fluor oder Chlor tragen können;

monocyclisches oder polycyclisches $C_3$-$C_{10}$-Cycloalkyl wie Cyclopropyl, Cyclobutyl, Cyclopentyl, Cyclohexyl, Cycloheptyl, Cyclooctyl, Menthyl, Norbornyl, Adamantyl, Tricyclodecanyl, vorzugsweise Cyclopropyl und Cyclohexyl; oder

monocyclisches oder polycyclisches $C_3$-$C_{10}$-Cycloalkylmethyl wie Cyclopropylmethyl, Cyclobutylmethyl, Cyclopentylmethyl, Cyclohexylmethyl, Cycloheptylmethyl, Cyclooctylmethyl, vorzugsweise Cyclopropylmethyl oder Cyclohexylmethyl;

wobei diese Ringe ein bis drei $C_1$-$C_4$-Alkylgruppen wie Methyl, Ethyl, Propyl, 1-Methylethyl, Butyl, 1-Methylpropyl, 2-Methylpropyl und 1,1-Dimethylethyl, vorzugsweise Methyl und Ethyl, insbesondere Methyl; oder einen Phenylring tragen können;

monocyclisches oder polycyclisches $C_5$-$C_{10}$-Cycloalkenyl wie vorzugsweise 2-Cyclohexen-1-yl;

oder monocyclisches oder polycyclisches $C_5$-$C_{10}$-Cycloalkenylmethyl wie vorzugsweise 1-Cyclohexenylmethyl, 2-Cyclohexenylmethyl und 3-Cyclohexenylmethyl;

wobei diese Ringe ein bis drei $C_1$-$C_4$-Alkylgruppen wie Methyl, Ethyl, Propyl, 1-Methylethyl, Butyl, 1-Methylpropyl, 2-Methylpropyl und 1,1-Dimethylethyl, vorzugsweise Methyl;

oder einen Phenylring tragen können;

Phenyl, Phenyl-$C_1$-$C_4$-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-1-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl und 1,1-Dimethyl-2-phenylethyl, vorzugsweise Benzyl und 2-Phenylethyl;

oder

Phenyl-$C_2$-$C_6$-alkenyl, besonders Phenyl-$C_2$-$C_4$-alkenyl wie 2-Phenylethenyl, 2-Phenyl-1-propenyl, 2-Phenyl-2-propenyl, 2-Phenyl-1-methylethenyl, 2-Phenyl-1-butenyl, 2-Phenyl-2-butenyl, 2-Phenyl-3-butenyl, 2-Phenyl-1-methyl-1-propenyl und 2-Phenyl-1-methyl-2-propenyl;

wobei die aromatischen Reste ein bis fünf Halogenatome wie Fluor, Chlor, Brom und Jod, vorzugsweise Fluor, Chlor und Brom, insbesondere Fluor und Chlor und/oder ein bis drei der folgenden Gruppen tragen können:

Nitro, $C_1$-$C_4$-Aklyl wie Methyl, Ethyl, Propyl, 1-Methylethyl, Butyl, 1-Methylpropyl, 2-Methylpropyl und 1,1-Dimethylethyl, vorzugsweise Methyl, 1-Methylethyl und 1,1-Dimethylethyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy;

$C_1$-$C_4$-Halogenalkoxy, besonders $C_1$-$C_2$-Halogenalkoxy wie Chlormethyloxy, Dichlormethyloxy, Trichlormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio;

und $C_1$-$C_4$-Halogenalkylthio, besonders $C_1$-$C_2$-Halogenalkylthio wie Chlormethylthio, Dichlormethylthio, Trichlormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio.

Im Hinblick auf die bestimmungsgemäße Verwendung in wachstumsregulierenden Mitteln sind Verbindungen IA besonders bevorzugt, in denen $R^1$ folgende Bedeutung hat:

Methyl, Ethyl, Propyl, 1-Methylethyl, Butyl, 1-Methyl-propyl, 2-Methyl-propyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl,3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl, Butyl, 1-Methyl-propyl, 2-Methylpropyl und 1,1-Dimethylethyl, 2-Propenyl, 2-Butenyl, 3-Butenyl, 1-Methyl-2-propenyl, 2-Methyl-2-propenyl, 3-Methyl-2-butenyl oder Benzyl, wobei diese Gruppen ein bis fünf Halogenatome wie Fluor, Chlor, Brom und Jod, vorzugsweise Fluor und Chlor tragen können.

Beispiele für insbesondere bevorzugte Oxalyl-bishydroxamsäurederivate der allgemeinen Formel IA sind in der folgenden Tabelle aufgeführt.

Tabelle

$R^1$-ONH-CO-CO-NHO-$R^2$ (I)

| $R^1$ | $R^2$ |
|---|---|
| $CH_3$ | $CH_3$ |
| $CH_3$ | $C_2H_5$ |
| $CH_3$ | n-$C_3H_7$ |
| $CH_3$ | i-$C_3H_7$ |
| $CH_3$ | n-$C_4H_9$ |
| $CH_3$ | i-$C_4H_9$ |
| $CH_3$ | sek.-$C_4H_9$ |
| $CH_3$ | n-$C_5H_{11}$ |
| $CH_3$ | i-$C_5H_{11}$ |
| $CH_3$ | n-$C_6H_{13}$ |
| $CH_3$ | $(CH_3)_2CH-CH_2-CH_2-CH_2-$ |
| $CH_3$ | n-$C_7H_{15}$ |
| $CH_3$ | n-$C_8H_{17}$ |
| $CH_3$ | $CH_3CH_2CH_2CH_2CH(C_2H_5)CH_2-$ |
| $CH_3$ | n-$C_9H_{19}$ |
| $CH_3$ | n-$C_{10}H_{21}$ |
| $CH_3$ | n-$C_{12}H_{25}$ |
| $CH_3$ | n-$C_{14}H_{29}$ |
| $CH_3$ | n-$C_{16}H_{33}$ |
| $CH_3$ | n-$C_{18}H_{37}$ |
| $CH_3$ | n-$C_{20}H_{41}$ |
| $CH_3$ | Cyclopropylmethyl |
| $CH_3$ | Cyclopentyl |
| $CH_3$ | Cyclohexyl |
| $CH_3$ | Cyclohexylmethyl |
| $CH_3$ | 4-Methylcyclohexyl |
| $CH_3$ | 4-Ethylcyclohexyl |
| $CH_3$ | 4-tert.-Butylcyclohexyl |
| $CH_3$ | Allyl |
| $CH_3$ | $CH_3CH=CHCH_2-$ |
| $CH_3$ | $(CH_3)_2C=CHCH_2-$ |
| $CH_3$ | Geranyl |
| $CH_3$ | $HC=CCH_2-$ |
| $CH_3$ | $CH_3C=CCH_2$ |
| $CH_3$ | $ClCH_2=CHCH_2-$ |

Tabelle (Fortsetzung)

| $R^1$ | $R^2$ |
|---|---|
| $CH_3$ | $CH_2=C(Cl)CH_2-$ |
| $CH_3$ | $CH_2=C(Br)CH_2-$ |
| $CH_3$ | $ClCH=C(Cl)CH_2-$ |
| $CH_3$ | $Cl_2=C(Cl)CH_2-$ |
| $CH_3$ | $ClCH_2CH_2-$ |
| $CH_3$ | $Cl_3CCH_2-$ |
| $CH_3$ | $ClCH_2CH_2CH_2-$ |
| $CH_3$ | $Cl(CH_2)_4-$ |
| $CH_3$ | $BrCH_2CH_2-$ |
| $CH_3$ | $Cl-(CH_2)_6-$ |
| $CH_3$ | $Cl-(CH_2)_8-$ |
| $CH_3$ | $Br-(CH_2)_4-$ |
| $CH_3$ | $Br-(CH_2)_6-$ |
| $CH_3$ | $C_6H_5-CH_2-$ |
| $CH_3$ | $4Cl-C_6H_4-CH_2-$ |
| $CH_3$ | $3Cl-C_6H_4-CH_2-$ |
| $CH_3$ | $3,4Cl_2-C_6H_3-CH_2-$ |
| $CH_3$ | $2,4Cl_2-C_6H_3-CH_2-$ |
| $CH_3$ | $4F-C_6H_4-CH_2-$ |
| $CH_3$ | $4Br-C_6H_4-CH_2-$ |
| $CH_3$ | $4-CH_3-C_6H_4-CH_2-$ |
| $CH_3$ | $2,4-(CH_3)_2-C_6H_3-CH_2-$ |
| $CH_3$ | $4-C_2H_5-C_6H_4-CH_2-$ |
| $CH_3$ | $4-C_3H_7-C_6H_4-CH_2-$ |
| $CH_3$ | $4-tert.-C_4H_9-C_6H_4-CH_2-$ |
| $CH_3$ | $4-CH_3O-C_6H_4-CH_2-$ |
| $CH_3$ | $4-O_2N-C_6H_4-CH_2-$ |
| $CH_3$ | $C_6H_5-CH_2CH_2-$ |
| $C_2H_5$ | $C_2H_5$ |
| $C_2H_5$ | $n-C_3H_9$ |
| $C_2H_5$ | $i-C_3H_9$ |
| $C_2H_5$ | $n-C_4H_9$ |
| $C_2H_5$ | sek.-$C_4H_9$ |
| $C_2H_5$ | $i-C_4H_9$ |
| $C_2H_5$ | $H_2C=C(CH_3)-CH_2-$ |
| $i-C_3H_7$ | $i-C_3H_7$ |
| Allyl | Allyl |

Tabelle (Fortsetzung)

| $R^1$ | $R^2$ |
|---|---|
| $H_2C=CClCH_2-$ | $H_2C=CClCH_2-$ |
| $H_2C=CBrCH_2-$ | $H_2C=CBrCH_2-$ |
| $ClCH=CHCH_2-$ | $ClCH=CHCH_2-$ |
| $CH_3CH=CHCH_2-$ | $CH_3CH=CHCH_2-$ |
| $H_2C=C(CH_3)CH_2-$ | $H_2C=C(CH_3)CH_2-$ |
| $ClCH_2CH_2CH_2-$ | $ClCH_2CH_2CH_2-$ |
| $n-C_4H_9$ | $n-C_4H_9$ |
| $i-C_4H_9$ | $i-C_4H_9$ |
| $sek.-C_4H_9$ | $sek.-C_4H_9$ |
| $n-C_5H_{11}$ | $n-C_5H_{11}-$ |
| $n-C_6H_{13}$ | $n-C_6H_{13}-$ |
| $HC\equiv CCH_2-$ | $HC\equiv CCH_2-$ |
| $C_6H_5-CH_2-$ | $C_6H_5-CH_2-$ |

Als Salze der Wirkstoffe der Formel IA kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen wachstumsregulierenden Wirkstoffe IA bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Wirkstoffe IA eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen

Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Wirkstoffe IA können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.006 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.010 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0, 1 Gew.% des Wirkstoffs enthält.

VI. 3 Gewichtsteile des Wirkstoffs Nr. 1.013 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.009 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.015 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 90 Gewichtsteile der Verbindung Nr. 1.011 mit 10 Gewichtsteilen N-Methyl-α-pyrrolidon und erhält eine Lösung, die zur Anwendung in Form kleinster Tropfen geeignet ist.

X. 20 Gewichtsteile der Verbindung Nr. 1.005 werden in einer Mischung gelöst, die aus 80 Gewichtsteilen xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

XI. 20 Gewichtsteile der Verbindung Nr. 1.004 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XII. 20 Gewichtsteile des Wirkstoffs Nr. 1.007 werden in einer Mischung gelöst, die aus 25 Gewichtsteilen

Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XIII. 20 Gewichtsteile des Wirkstoffs Nr. 1.012 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

XIV. 3 Gewichtsteile des Wirkstoffs Nr. 1.015 werden mit 97 Gewichtsteilen feinteiligem Kaolin vermischt. Man erhält auf diese Weise ein Stäubemittel, das 3 Gew.% des Wirkstoffs enthält.

XV. 30 Gewichtsteile des Wirkstoffs Nr. 1.005 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

XVI. 20 Gewichtsteile des Wirkstoffs Nr. 1.006 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenolsulfonsäure-Formaldehyd-Kondensates und 68 Gewichtsteilen eines paraffinischen Mineralöls innig vermischt. Man erhält eine stabile ölige Dispersion.

Die Applikation der wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 10, vorzugsweise 0,01 bis 5 kg/ha, insbesondere 0,05 bis 2 kg/ha aktive Substanz (a.S.).

Die Verbindungen der Formel IA können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel IA im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel IA Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel IA können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemäßen Verbindungen bzw. sie enthaltende Mittel in einer großen Zahl von Kulturpflanzen eingesetzt werden.

Zur Verbreiterung des Wirkungsspektrums und zur Erzielung synergistischer Effekte können die erfindungsgemäßen Wirkstoffe IA mit zahlreichen Vertretern herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Wirkstoffe IA allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die

Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Wirkstoffe IA benutzt. Die so erhaltenen Verbindungen sind in den nachstehenden Tabellen mit physikalischen Angaben aufgeführt.

Beispiel 1

Bis(propoxyamino)ethandion

200 ml Essigsäure wurden bei 20°C nacheinander mit 17,5 g (0,157 Mol) O-Propylhydroxylamin-hydrochlorid, 18,1 g (0,22 Mol) Natriumacetat und 10 g (0,0787 Mol) Oxalylchlorid versetzt. Nach zwanzigstündigem Nachrühren bei 25°C wurde das Gemisch abgesaugt und das Filtrat im Vakuum eingeengt. Der Rückstand wurde mit 200 ml Essigester versetzt, dreimal mit je 20 ml Wasser gewaschen, über $Na_2SO_4$ getrocknet, im Vakuum eingeengt und anschließend in 40 ml 1:1 Ethylether:Pentan kristallisiert. Man erhielt 8,7 g (54,2 % der Theorie) Bis(propoxyamino)ethandion als farblose Kristalle vom Schmp. 147-150°C (Wirkstoffbeispiel 1.008).

Beispiel 2

1-(Propoxyamino)-2-allyloxyamino)ethandion

100 ml Methanol wurden nacheinander mit 16,1 g (0,1 Mol) (Propoxyamino)oxoessigsäuremethylester, 16,5 g (0,15 Mol) O-Allylhydroxylamin-hydrochlorid und 15 g (0,15 Mol) Triethylamin versetzt. Das Gemisch wurde 20 Stunden bei 60°C nachgerührt und anschließend eingeengt. Der Rückstand wurde mit 250 ml Methylenchlorid versetzt, zweimal mit je 50 ml Wasser gewaschen, über $Na_2SO_4$ getrocknet und im Vakuum eingeengt. Der Rückstand wurde mit 40 ml 1:1 Ethylether:Pentan versetzt und die Kristalle abgesaugt und getrocknet. Man erhielt 12 g (59,4 % der Theorie) 1-(Propoxyamino)-2-(allyloxyamino)ethandion als weiße Kristalle vom Schmp. 90-91°C (Wirkstoffbeispiel 1.009).

Tabelle 1

$$R^1\text{-O-NH-CO-CO-NHO-}R^2 \quad (I)$$

| Beispiel Nr. | $R^1$ | $R^2$ | Fp (°C) Sdp. (°C/mbar) |
|---|---|---|---|
| 1.001 | $CH_3$ | $CH_3$ | 166-168 |
| 1.002 | $C_2H_5$ | $C_2H_5$ | 183-185* |
| 1.003 | $H_2C{=}CBr{-}CH_2{-}$ | $H_2C{=}CBr{-}CH_2{-}$ | 112-114 |
| 1.004 | $CH_3{-}CH{=}CH{-}CH_2{-}$ | $CH_3{-}CH{=}CH{-}CH_2{-}$ | 190-192 |
| 1.005 | $H_2C{=}C(CH_3){-}CH_2{-}$ | $H_2C{=}C(CH_3){-}CH_2{-}$ | 107-110 |
| 1.006 | $n{-}C_4H_9$ | $n{-}C_4H_9$ | 151-153 |
| 1.007 | $HC{\equiv}C{-}CH_2{-}$ | $HC{\equiv}C{-}CH_2{-}$ | 195-197 |
| 1.008 | $CH_3CH_2CH_2{-}$ | $CH_3CH_2CH_2{-}$ | 147-150 |
| 1.009 | $CH_3CH_2CH_2{-}$ | $CH_2{=}CHCH_2{-}$ | 90- 91 |
| 1.010 | $CH_2{=}CHCH_2$ | $CH_2{=}CHCH_2$ | 191-193 |
| 1.011 | $i{-}C_3H_7$ | $i{-}C_3H_7$ | 167-169 |
| 1.012 | $ClCH{=}CHCH_2$ | $ClCH{=}CHCH_2$ | 198-201 |
| 1.013 | $n{-}C_5H_{11}$ | $n{-}C_5H_{11}$ | 138-140 |
| 1.014 | $n{-}C_6H_{13}$ | $n{-}C_6H_{13}$ | 145-148 |
| 1.015 | $C_6H_5CH_2$ | $C_6H_5CH_2$ | 195-196 |

* W. Lossen et al., Chem.Ber. 27, 1111 (1894)

Anwendungsbeispiele

Die wachstumsregulierende Wirkung der Verbindungen der allgemeinen Formel I ließ sich durch folgende Versuche zeigen:

Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a) bzw. mit Wasser im Fall von b) verdünnt.

Als Kulturgefäße dienten Plastikblumentöpfe (Durchmesser ca. 12,5 cm; Volumen ca. 500 ml) mit lehmigem Sand mit etwa 3,0 % Humus als Substrat. Die Samen der Testpflanzen wurden nach Arten getrennt eingesät.

Bei Vorauflaufanwendung wurden die in Wasser suspendierten oder emulgierten Wirkstoffe direkt nach der Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Die Abdeckung bewirkt ein gleichmäßiges Keimen der Testpflanzen.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Die beobachtete wachstumsregulierende Wirkung wurde bei Versuchsende durch die Messung der Wuchshöhe belegt. Die so gewonnenen Meßwerte wurden zur Wuchshöhe von nicht behandelten Pflanzen in Relation gesetzt. Als Vergleichsubstanz zur Beurteilung der wachstumsregulierenden Wirkung diente N-(2-Chlorethyl)-N,N,N-trimethyl-ammonium chlorid (Beispiel A).

Die in den Gewächshausversuchen verwendeten Pflanzen waren: Sommerweizen (cv. "Ralle"), Sommergerste (cv. "Aramir"), Reis (cv. "Bahia"), Sonnenblumen (cv. "Spanners Allzweck") und Sommerraps (cv. "Petranova").

Bei Nachauflaufanwendung von 0,4 mg/Gefäß zeigte die Verbindung 1.005 bei Sommerweizen (cv. "Ralle"), bei Sommergerste (cv. "Aramir") und bei Reis (cv. "Bahia") eine bessere Wirkung als der bekannte Wachstumsregulator A.

Bei Nachauflaufanwendung von 6 mg/Gefäß zeigte die Verbindung 1.005 bei Sonnenblumen (cv. "Spanners Allzweck") und bei Sommerraps (cv. "Petranova") eine bessere Wirkung als der bekannte Wachstumsregulator A.

Gleichlaufend zu der Reduzierung des Längenwachstums stieg die Farbintensität der Blätter an. Der erhöhte Chlorophyllgehalt läßt eine erhöhte Photosyntheserate und damit eine Steigerung des Ertrags erwarten.

## Patentansprüche

1. Oxalyl-bishydroxamsäurederivate der allgemeinen Formel I,

$$R^1\text{-ONH-CO-CO-NHO-}R^2 \qquad I$$

in der die Substituenten folgende Bedeutung haben:
$R^1$ und $R^2$ unabhängig voneinander
$C_1$-$C_{20}$-Alkyl, $C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen oder einen Phenylring tragen können;
monocyclisches oder polycyclisches $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Phenylring tragen können;
Phenyl, Phenyl-$C_1$-$C_4$-alkyl oder Phenyl-$C_2$-$C_6$-alkenyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio,
wobei $R^1$ und $R^2$ nicht gleichzeitig Methyl oder Ethyl bedeuten sowie deren landwirtschaftlich brauchbaren Salze.

2. Verfahren zur Herstellung der Verbindungen der Formel I gemäß Anspruch 1, dadurch gekennzeichnet, daß man einen Oxalylhydroxamsäureester der allgemeinen Formel II,

$$R^1\text{-ONH-CO-CO-OCH}_3 \qquad II$$

in an sich bekannter Weise in einem inerten organischen Lösungsmittel mit einem Hydroxylaminderivat der allgemeinen Formel III,

$$R^2\text{-ONH}_2 \qquad III$$

umsetzt.

3. Verfahren zur Herstellung der Verbindungen der Formel I gemäß Anspruch 1, in denen $R^1$ und $R^2$ die gleiche Bedeutung haben, dadurch gekennzeichnet, daß man ein Oxalylhalogenid der allgemeinen Formel IV,

$$\text{Hal-CO-CO-Hal} \qquad IV$$

in der Hal für Halogenatome steht, in an sich bekannter Weise in einem inerten organischen Lösungsmittel in Gegenwart eine Base mit einem Hydroxylamin der allgemeinen Formel III

$$R^1\text{-ONH}_2 \qquad III$$

umsetzt.

4. Mittel zur Regulierung des Pflanzenwachstums, enthaltend eine regulativ wirksame Menge einer Verbindung der allgemeinen Formel I gemäß Anspruch 1 und inerte Zusatzstoffe.

5. Verfahren zu Regulierung des Pflanzenwachstums, dadurch gekennzeichnet, daß man die Pflanzen, ihren Lebensraum und/oder ihre Samen mit einer regulativ wirksamen Menge einer Oxalyl-bishydroxamsäure der allgemeinen Formel IA,

$$R^1\text{-ONH-CO-CO-NHO-}R^2 \qquad IA$$

in der die Substituenten folgende Bedeutung haben:
$R^1$ und $R^2$ unabhängig voneinander
$C_1$-$C_{20}$-Alkyl, $C_3$-$C_{18}$-Alkenyl oder $C_3$-$C_8$-Alkinyl, wobei diese Gruppen ein bis fünf Halogenatome tragen

können;
monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen oder einen Phenylring tragen können;
monocyclisches oder polycyclisches $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Phenylring tragen können;
Phenyl, Phenyl-$C_1$-$C_4$-alkyl oder Phenyl-$C_2$-$C_6$-alkenyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio,
oder deren landwirtschaftlich brauchbaren Salze behandelt.

## Claims

**1.** An oxalylbishydroxamic acid derivative of the formula I

$$R^1\text{-ONH-CO-CO-NHO-}R^2 \qquad \text{I}$$

where $R^1$ and $R^2$ independently of one another are each $C_1$-$C_{20}$-alkyl, $C_3$-$C_{18}$-alkenyl or $C_3$-$C_8$-alkynyl, where these groups may carry from one to five halogen atoms,
monocyclic or polycyclic $C_3$-$C_{10}$-cycloalkyl or $C_3$-$C_{10}$-cycloalkylmethyl, where these rings may carry from one to three $C_1$-$C_4$-alkyl groups or one phenyl ring,
monocyclic or polycyclic $C_5$-$C_{10}$-cycloalkenyl or $C_5$-$C_{10}$-cycloalkenylmethyl, where these rings may carry from one to three $C_1$-$C_4$-alkyl groups or one phenyl ring, and
phenyl, phenyl-$C_1$-$C_4$-alkyl or phenyl-$C_2$-$C_6$-alkenyl, where the aromatic radicals may carry from one to five halogen atoms and/or from one to three of the following groups: nitro, $C_1$-$C_4$-aklyl [sic], $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,
where $R^1$ and $R^2$ are not simultaneously methyl or ethyl, and its agriculturally useful salts.

**2.** A process for the preparation of a compound of the formula I as claimed in claim 1, wherein an oxalylhydroxamic ester of the formula II

$$R^1\text{-ONH-CO-CO-OCH}_3 \qquad \text{II}$$

is reacted with a hydroxylamine derivative of the formula III

$$R^2\text{-ONH}_2 \qquad \text{III}$$

in a conventional manner in an inert organic solvent.

**3.** A process for the preparation of a compound of the formula I as claimed in claim 1, in which $R^1$ and $R^2$ have the same meanings, wherein an oxalyl halide of the formula IV

$$\text{Hal-CO-CO-Hal} \qquad \text{IV}$$

where Hal is halogen, is reacted in a conventional manner in an inert organic solvent in the presence of a base with hydroxylamine of the formula III

$$R^1\text{-ONH}_2 \qquad \text{III}$$

**4.** A plant growth regulator containing an amount, having a regulating effect, of a compound of the formula I as claimed in claim 1 and inert additives.

**5.** A method for regulating plant growth, wherein the plants, their habitat or their seeds are treated with an amount, having a regulating effect, of an oxalylbishydroxamic acid of the formula IA

$$R^1\text{-ONH-CO-CO-NHO-}R^2 \qquad \text{IA}$$

where $R^1$ and $R^2$ independently of one another are each $C_1$-$C_{20}$-alkyl, $C_3$-$C_{18}$-alkenyl or $C_3$-$C_8$-alkynyl, where these groups may carry from one to five halogen atoms,
monocyclic or polycyclic $C_3$-$C_{10}$-cycloalkyl or $C_3$-$C_{10}$-cycloalkylmethyl, where these rings may carry from one to three $C_1$-$C_4$-alkyl groups or one phenyl ring,
monocyclic or polycyclic $C_5$-$C_{10}$-cycloalkenyl or $C_5$-$C_{10}$-cycloalkenylmethyl, where these rings may carry from one to three $C_1$-$C_4$-alkyl groups or one phenyl ring, and
phenyl, phenyl-$C_1$-$C_4$-alkyl or phenyl-$C_2$-$C_6$-alkenyl, where the aromatic radicals may carry from one to five halogen atoms and/or from one to three of the following groups: nitro, $C_1$-$C_4$-aklyl [sic], $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,
and their agriculturally useful salts.

## Revendications

**1.** Dérivés d'acides oxalyl-bis-hydroxamiques de formule générale I

$R^1$-ONH-CO-CO-NHO-$R^2$ I

dans laquelle les symboles ont les significations suivantes :

$R^1$ et $R^2$ représentent chacun, indépendamment l'un de l'autre, un groupe alkyle en $C_1$-$C_{20}$, alcényle en $C_3$-$C_{18}$ ou alcynyle en $C_3$-$C_8$, ces groupes pouvant porter 1 à 5 atomes d'halogènes ;

un groupe cycloalkyle en $C_3$-$C_{10}$ ou cycloalkylméthyle en $C_3$-$C_{10}$ monocyclique ou polycyclique, ces cycles pouvant porter 1 à 3 groupes alkyle en $C_1$-$C_4$ ou un cycle phényle ;

un groupe cycloalcényle en $C_5$-$C_{10}$ ou cyclalcénylméthyle en $C_5$-$C_{10}$ monocyclique ou polycyclique, ces cycles pouvant porter de 1 à 3 groupes alkyle en $C_1$-$C_4$ ou un cycle phényle ;

un groupe phényle, phényl-alkyle en $C_1$-$C_4$ ou phényl-alcényle en $C_2$-$C_6$ dont les parties aromatiques peuvent porter 1 à 5 atomes d'halogènes et/ou 1 à 3 atomes des groupes suivants : nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ ou halogénoalkylthio en $C_1$-$C_4$,

sous réserve que $R^1$ et $R^2$ ne peuvent représenter tous deux simultanément des groupes méthyle ou éthyle,

et leurs sels acceptables pour des usages agricoles.

**2.** Procédé de préparation des composés de formule I de la revendication 1, caractérisé en ce que l'on fait réagir un ester d'acide oxalylhydroxyamique de formule générale II

$R^1$-ONH-CO-CO-$OCH_3$ II

de manière connue en soi, dans un solvant organique inerte, avec un dérivé de l'hydroxylamine de formule générale III

$R^2$-$ONH_2$ III

**3.** Procédé de préparation des composés de formule I de la revendication 1 pour lesquels $R^1$ et $R^2$ ont la même signification, caractérisé en ce que l'on fait réagir un halogénure d'oxalyle de formule générale IV

Hal-CO-CO-Hal IV

dans laquelle les symboles Hal représentent des atomes d'halogènes, de manière connue en soi, dans un solvant organique inerte, en présence d'une base, avec une hydroxylamine de formule générale III

$R^1$-$ONH_2$ III

**4.** Produit pour la régulation de la croissance des végétaux, contenant une quantité régulatrice efficace d'un composé de formule générale I, selon la revendication 1 et des additifs inertes.

**5.** Procédé pour la régulation de la croissance des végétaux, caractérisé en ce que l'on traite les végétaux, leur habitat et/ou leurs semences par une quantité régulatrice efficace d'un acide oxalyl-bishydroxamique de formule générale IA

$R^1$-ONH-CO-CO-NHO-$R^2$ IA

dans laquelle les symboles ont les significations suivantes :

$R^1$ et $R^2$ représentent chacun, indépendamment l'un de l'autre, un groupe alkyle en $C_1$-$C_{20}$, alcényle en $C_3$-$C_{18}$ ou alcynyle en $C_3$-$C_8$, ces groupes pouvant porter 1 à 5 atomes d'halogènes ;

un groupe cycloalkyle en $C_3$-$C_{10}$ ou cycloalkylméthyle en $C_3$-$C_{10}$ monocyclique ou polycyclique, ces cycles pouvant porter 1 à 3 groupes alkyle en $C_1$-$C_4$ ou un cycle phényle ;

un groupe cycloalcényle en $C_5$-$C_{10}$ ou cycloalcénylméthyle en $C_5$-$C_{10}$ monocyclique ou polycyclique, ces cycles pouvant porter 1 à 3 groupes alkyle en $C_1$-$C_4$ ou un cycle phényle ;

un groupe phényle, phényl-alkyle en $C_1$-$C_4$ ou phényl-alcényle en $C_2$-$C_6$ dont les parties aromatiques peuvent porter 1 à 5 atomes d'halogènes et/ou 1 à 3 des groupes suivants : nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ ou halogénoalkylthio en $C_1$-$C_4$,

ou de leurs sels acceptables pour les usages agricoles.